# FASCICULE DE BREVET EUROPEEN

(11) **EP 3 129 461 B1**
(45) Date de publication et mention de la délivrance du brevet: **03.06.2020**
(21) Numéro de dépôt: 15720358.9
(22) Date de dépôt: 07.04.2015
(51) Int. Cl.: C12M 1/34

(54) **DISPOSITIF D'INCUBATION ET DE DETECTION**
INKUBATIONS- UND DETEKTIONSVORRICHTUNG
INCUBATION AND DETECTION DEVICE

(30) Priorité: 07.04.2014 FR 1453047; 28.08.2014 FR 1458040
(43) Date de publication de la demande: 15.02.2017
(73) Titulaire: bioMérieux, 69280 Marcy l'Etoile (FR)
(72) Inventeur: BARLET, Pierre, F-67000 Strasbourg (FR); SALA, Guillaume, 67150 Schaeffersheim (FR); PIERQUIN, Joseph, F-67120 Molsheim (FR)
(74) Mandataire: Cabinet Bleger-Rhein-Poupon
(86) Numéro de dépôt international: PCT/FR2015/050904
(87) Numéro de publication internationale: WO 2015/155468

(56) Documents cités:
- WO-A1-2013/110734
- GB-A- 2 494 202
- DATABASE WPI Week 201056 Thomson Scientific, London, GB; AN 2010-J89042 XP002736067, & JP 2010 161978 A (GH KIMI GA FUCHIGAKUEN) 29 juillet 2010 (2010-07-29)

## Description

La présente invention concerne le domaine de la détection de micro-organismes en phase de croissance.

La présente invention trouvera son application principalement dans les domaines de la microbiologie industrielle et clinique, par exemple dans les industries pharmaceutiques, biotechnologiques, agroalimentaires ou encore les hôpitaux ou les laboratoires d'analyses médicales.

L'invention concerne plus particulièrement un dispositif permettant une incubation et une détection rapide de la formation de colonies, à partir de micro-organismes présents dans un échantillon, à la surface d'une membrane ou d'un milieu de culture solide ou semi-solide.

De nombreuses techniques sont mises en œuvres, à l'heure actuelle, pour permettre la détection de contaminants, par exemple de bactéries, dans un échantillon qui doit être testé.

La méthode la plus classique et la plus ancienne consiste en un dépôt à la surface d'un milieu de croissance gélosé, ce dernier pouvant être plus ou moins sélectif d'un ou plusieurs types de micro-organismes.

Le milieu est ensuite incubé à la température adéquate pour la croissance du ou des micro-organisme(s) recherché(s), pendant une durée pouvant aller jusqu'à plusieurs jours.

Une telle méthode présente l'inconvénient de nécessiter une période d'incubation relativement longue pour permettre une détection à l'œil nu des colonies qui se sont formées sur le support de culture.

Il est aussi connu de l'état de la technique de réaliser une réaction de polymérisation en chaine, également appelée amplification PCR, pour déterminer la présence de micro-organismes spécifiques au sein d'un échantillon, par amplification d'une séquence d'ADN ou d'ARN.

Ces méthodes présentent l'inconvénient de nécessiter plusieurs brins d'ADN c'est-à-dire plusieurs micro-organismes contaminants, généralement au moins plusieurs dizaines de micro-organismes. De telles méthodes sont souvent donc moins sensibles que les méthodes basées sur la croissance.

On connait encore la possibilité d'utiliser des techniques consistant à marquer les micro-organismes de manière à accentuer le contraste entre la lumière émise par les micro-organismes et celle émise par le support de croissance. L'utilisation de marqueurs de viabilité fluorescents comme la CFDA (diacétate de carboxyfluorescéine) ou non fluorescent comme le TTC (chlorure de tetrazolium), ou encore d'enzymes permettant de révéler la bioluminescence émise par exemple par l'ATP (adénosine tri-phosphate), permettent de détecter de manière précoce les micro-organismes, grâce à l'utilisation de systèmes optiques sensibles aux caractéristiques de la lumière émise, par exemple la longueur d'onde ou l'intensité.

On connait ainsi notamment la demande de brevet étasunienne US 2003/0155528 décrivant une méthode de détection de micro-organismes dans laquelle ces derniers sont marqués par l'intermédiaire de réactifs fluorescents appropriés (comme la fluorescéine) permettant de déterminer d'une part la quantité de micro-organismes et, d'autre part, de juger s'il s'agit de cellules viables ou mortes.

Cependant, ces techniques peuvent s'avérer lourdes à mettre en œuvre et nécessitent l'emploi de réactifs souvent coûteux et la présence d'une main d'œuvre qualifiée. De plus, elles se prêtent mal à la détection de contaminants sur un grand nombre d'échantillons, l'opération de marquage étant souvent difficilement automatisable. En outre, ces techniques présentent un risque de contamination de l'échantillon. En effet, l'ajout de réactifs nécessite une mise en contact dudit réactif avec les micro-organismes à détecter et s'opère généralement en ouvrant la boite contenant le milieu gélosé. Enfin, la mise en contact du réactif avec les micro-organismes présente le risque de destruction des cellules vivantes formant une colonie, surtout lorsque celles-ci sont à des stades précoces de croissances (comptant moins de 100 cellules typiquement).

On connait également, dans l'état de la technique, des méthodes basées sur une utilisation des propriétés lumineuses émises naturellement par les micro-organismes, en détectant par exemple l'auto-fluorescence desdits micro-organismes. Ainsi, il est possible de faciliter la distinction de colonies en utilisant le contraste existant entre la fluorescence naturelle émise par lesdits micro-organismes et le support, non fluorescent, sur lequel ils ont été déposés.

Une méthode utilisant ce principe est notamment décrite dans la demande internationale de brevet WO 03/022999, dans laquelle on utilise certaines propriétés optiques des colonies, comme l'auto-fluorescence.

Ces techniques permettent certes de faciliter la détection des colonies ou des micro-organismes auto-fluorescents, ceux-ci présentant alors un meilleur contraste avec la membrane ou le milieu de culture. Cependant, le niveau de fluorescence généré naturellement est de faible amplitude ce qui ne permet pas d'obtenir des temps de détection rapides par rapport à un marquage avec un fluorophore spécifique par exemple. De plus, l'émission parasite de fluorescence naturelle par le milieu de culture ou par d'autres particules présentes dans l'environnement, telles que des poussières, des fibres de membranes ou des particules plastiques provenant du support, peut engendrer un résultat faussement positif.

Enfin des techniques utilisant des systèmes optiques à fort grossissement peuvent être utilisées pour visualiser des colonies à des stades précoces de développement : c'est le cas des microscopes par exemple. Néanmoins, ces dispositifs se limitent à une détection sur de faibles surfaces, inférieures à 1mm² en général. Ainsi la mise en œuvre de ce type de techniques pour la détection sur un ou plusieurs supports de détection, tels que des membranes ou des milieux gélosés, s'avère à la fois long, de l'ordre de plusieurs minutes par cm², et couteux, du fait de la nécessité d'utiliser des systèmes à balayage.

Il est également connu, de l'état de la technique, le document de brevet WO 2013/110734 qui propose un dispositif permettant une détection précoce, et pouvant être automatisée, de l'apparition de colonies à la surface d'un support de croissance, notamment une membrane ou un milieu de culture gélosé.

Plus particulièrement, dans ce dispositif, on retrouve une surface de détection sur laquelle repose un support de croissance, par exemple une membrane ou un milieu gélosé, et un système de détection, de type scanner linéaire. Ce système comporte au moins un capteur CCD associé à un système optique.

Ce dispositif est particulièrement intéressant car il permet notamment de s'affranchir de l'utilisation de matériel optique coûteux ou de réactifs. En outre, un tel dispositif permet d'éviter toute contamination par d'autres micro-organismes ou particules, car les échantillons n'ont pas besoin d'être déplacés pendant l'incubation, étant donné que c'est le système de détection qui est mobile.

Toutefois, on s'est aperçu qu'un tel dispositif de détection, très performant en soi, pouvait encore être amélioré dans ses performances. On a par ailleurs constaté que les aménagements apportés à un tel dispositif de détection pouvaient également améliorer les performances des dispositifs d'incubation et de détection n'utilisant pas de scanner linéaire par exemple. GB2494202 divulgue un dispositif d'incubation et de détection de colonies. Pour réduire la formation de condensation sur le couvercle du réceptacle de culture, GB2494202 décrit la formation d'un gradient de température entre le haut et le bas du réceptacle de culture. Le couvercle transparent est chauffé un petit peu plus que le support de culture.

A cet effet, la présente invention concerne un dispositif d'incubation et de détection, rapide et sans mesure de fluorescence, de colonies issues de la multiplication de micro-organismes présents dans un échantillon à tester, comprenant :
- un moyen de chauffage,
- une surface de détection sur laquelle est disposé, immobile, au moins un réceptacle fermé d'un couvercle transparent dans lequel est placé un support de croissance des micro-organismes sous forme de colonies, ce support étant de type membrane ou milieu gélosé,
- un système de détection, comprenant un système optique permettant de visualiser lesdites colonies.

Ledit dispositif est caractérisé en ce qu'il comporte en outre un système de prévention de la formation de condensation dans ledit réceptacle, qui comprend un moyen de régulation de la température composé :
- d'un premier capteur de température opérant au-dessus dudit réceptacle,
- d'un second capteur de température opérant en dessous dudit réceptacle,
- dudit moyen de chauffage, qui agit au-dessus dudit réceptacle,
- d'un moyen de refroidissement agissant en-dessous dudit réceptacle, et
- des moyens de gestion aptes à collecter et analyser les informations recueillies par lesdits moyens capteurs, et à commander lesdits moyens de chauffage et de refroidissement en sorte de générer la température d'incubation d'une part souhaitée, et d'appliquer en permanence un gradient de température à la surface dudit réceptacle d'autre part.

De manière avantageuse, mais non limitative, le système de détection est de type scanner linéaire, monté mobile et à plat pour balayer en tout ou partie ladite surface, comportant au moins un capteur CCD ou CMOS auquel est associé un système optique composé d' au moins un éclairage et d'au moins une optique, de type lentille.

La présence du système de prévention de formation de la condensation est particulièrement avantageuse.

En effet, il permet d'éviter que de la condensation ne se forme sur la partie du couvercle transparent, à l'intérieur du réceptacle lorsque celui-ci est fermé. Lors de l'incubation, des réceptacles, des gouttelettes d'eau sont en effet susceptibles de se former sur la surface du couvercle interne au réceptacle, localisée du côté du support de croissance, celui-ci pouvant consister en une membrane déposée sur un milieu gélosé ou en un milieu gélosé seul, par exemple.

La condensation peut s'avérer particulièrement problématique dans les systèmes de détection de colonies. En effet elle rend opaque le couvercle du réceptacle et empêche ainsi toute détection de la croissance microbienne.

Dans l'état de la technique, la solution retenue pour arriver à lire le résultat sur la gélose ou sur la membrane est d'ouvrir le réceptacle, en retirant le couvercle sur lequel se trouvent les gouttelettes de condensation. Toutefois, une telle solution ne peut être considérée comme adéquate, car elle est très fortement susceptible d'entrainer la contamination du milieu de culture placé dans le réceptacle, par des germes présents dans l'environnement. Il est alors possible d'obtenir un résultat faussement positif, ce qui peut s'avérer particulièrement problématique, voire dangereux, notamment en application clinique.

Le dispositif de détection selon la présente invention, tel que décrit ci-dessus, permet de résoudre ce problème.

En outre, le présent dispositif est particulièrement avantageux car il permet de combiner les fonctions d'incubation de l'échantillon et de détection de micro-organismes présents dans ledit échantillon.

Une telle combinaison présente de nombreux avantages.

En particulier, l'échantillon analysé est constamment maintenu à la bonne température, étant donné qu'il ne sort pas du dispositif, ce qui facilite la croissance des micro-organismes.

De plus, un tel dispositif permet d'éviter les erreurs de manipulation du réceptacle contenant le support de croissance, de telles erreurs étant toujours possibles avec un bras robotisé d'un dispositif automatisé de l'état de la technique. En conséquence, les risques de contamination de l'échantillon sont diminués voir annulés avec le dispositif selon l'invention.

Enfin, le dispositif associant incubation d'un échantillon et détection de micro-organismes participe également à la réduction de la condensation sur le couvercle du réceptacle.

Dans un mode de réalisation particulier, les moyens de gestion permettent d'appliquer un gradient de température d'au moins 0,10°C entre l'intérieur et l'extérieur du réceptacle.

Un tel gradient de température permet, de manière tout particulièrement optimale, d'éviter la formation de condensation au niveau du réceptacle.

On notera que préférentiellement, la mesure de température est réalisée au moyen d'une thermopile.

Le dispositif d'incubation et de détection selon l'invention comporte de préférence des moyens d'ajustement de la position du ou des réceptacles, et donc du ou des supports de croissance, par rapport au système optique.

Le dispositif d'incubation et de détection selon l'invention comporte également des moyens de correction automatique de la distance qui sépare le système optique du ou des réceptacles, et donc du ou des supports de croissance.

Un tel mode de réalisation permet un positionnement constant et précis du réceptacle par rapport au système optique du système de détection, ce qui va faciliter la détection des colonies qui se développent sur le support de croissance notamment en s'assurant que celui-ci est dans la zone de profondeur de champ du système optique.

D'autre part, ledit dispositif d'incubation et de détection comporte des moyens de correction de l'orientation du ou des réceptacles, et donc du ou des supports de croissance, par rapport à l'axe du système optique.

Cette caractéristique constitue un moyen particulièrement astucieux pour remédier aux phénomènes d'aberrations optiques qui sont susceptibles d'aboutir à la formation d'images qui sont déformées. De tels phénomènes sont dus à la structure du scanner. Il en résulte que les supports de croissance apparaissent ovales et certaines zones desdits supports ne sont alors pas visibles. En conséquence, le système de détection peut ne pas détecter certaines colonies et il existe donc, du fait des phénomènes d'aberrations optiques, un risque non négligeable d'obtenir un résultat faussement négatif.

Or, tout comme un résultat faussement positif, un faux-négatif peut être dangereux, et en particulier lorsqu'il s'agit de diagnostiquer un patient en microbiologie clinique ou de s'assurer de la stérilité de produits comme des médicaments en microbiologie industrielle.

Ainsi de manière avantageuse, dans le dispositif d'incubation et de détection selon l'invention, chacun du ou des réceptacles repose sur la surface de détection au travers de moyens de calage aptes à incliner ledit réceptacle et lui donner l'orientation la plus favorable par rapport au système optique, selon la position occupée par le réceptacle lors de l'opération de détection.

Dans un mode de réalisation avantageux, la surface de détection consiste en un tiroir, monté coulissant dans ledit dispositif, apte à passer d'une position ouverte, pour recevoir le ou les réceptacles fermés, à une position fermée, dans laquelle peut être réalisé un balayage du ou desdits réceptacles et donc du ou des supports de croissance par le système de détection, lesdits supports étant disposés directement dans ledit tiroir.

Plus avantageusement encore, le ou les réceptacles sont disposés dans le tiroir au travers d'un plateau amovible.

Ledit plateau amovible est avantageusement muni en périphérie et inférieurement de cales prévues aptes, lorsque le tiroir est en position fermée, à coopérer avec des butées de profondeur réglables que comporte intérieurement ledit dispositif, en sorte d'amener, par soulèvement, et de maintenir ledit plateau, et donc le ou les réceptacles qu'il porte, à la bonne distance du système optique.

Dans ce mode de réalisation particulier, où le plateau peut accueillir plusieurs réceptacles, la surface de détection sur laquelle sont disposés les réceptacles, comporte des emplacements dédiés précisément à ces réceptacles, le fond de chacun desdits emplacements présente une certaine inclinaison par rapport au plan général du plateau, en fonction du positionnement de ces emplacements sur ledit plateau, de sorte d'obtenir la correction souhaitée par rapport au système optique.

Une telle caractéristique permet de remédier aux phénomènes d'aberrations optiques de manière particulièrement satisfaisante.

Ainsi, de préférence, la surface de détection sur laquelle est disposé au moins un réceptacle, est configurée pour donner à chacun des réceptacles une inclinaison par rapport au système optique.

Enfin, de manière également avantageuse, le système optique du dispositif d'incubation et de détection selon l'invention, comporte un système de calibration, qui consiste en un élément amovible dans un but de nettoyage et/ou de remplacement.

En effet, les systèmes de détection, de type scanner linéaire, ont besoin, pour repérer et ajuster le niveau de blanc lors de la prise d'images, de prendre une image sur une bande blanche dite de calibration, intégrée au système de détection.

Dans le cas où cette bande de calibration est salie, par exemple par une poussière qui se déposerait dessus, cela a pour conséquence de détériorer la qualité des images prises par le scanner qui présentent des raies de couleurs verticales, empêchant ou retardant potentiellement la détection de micro-colonies localisées sur ces raies.

Une solution envisagée consiste à démonter la totalité de l'appareil afin de nettoyer la bande blanche de calibration. Toutefois, cette solution n'est pas satisfaisante, car elle est fastidieuse et particulièrement longue à effectuer. Une autre solution consiste tout simplement à remplacer l'appareil dans son intégralité, ce qui est coûteux et peu écologique.

La solution proposée dans le dispositif de détection selon l'invention permet de répondre au problème posé.

D'autres caractéristiques et avantages de l'invention ressortiront de la description détaillée des modes de réalisation non limitatifs de l'invention, en référence aux figures annexées dans lesquelles :
- la figure 1 représente schématiquement une vue en perspective d'un dispositif d'incubation et de détection selon l'invention.
- la figure 2 représente une vue en coupe selon un plan vertical du même dispositif de détection.
- la figure 3a représente une vue en perspective d'une partie du même dispositif de détection.
- la figure 3b représente une vue en plan de la même partie.
- la figure 3c représente une vue en coupe selon l'axe AA' de la figure 3b.

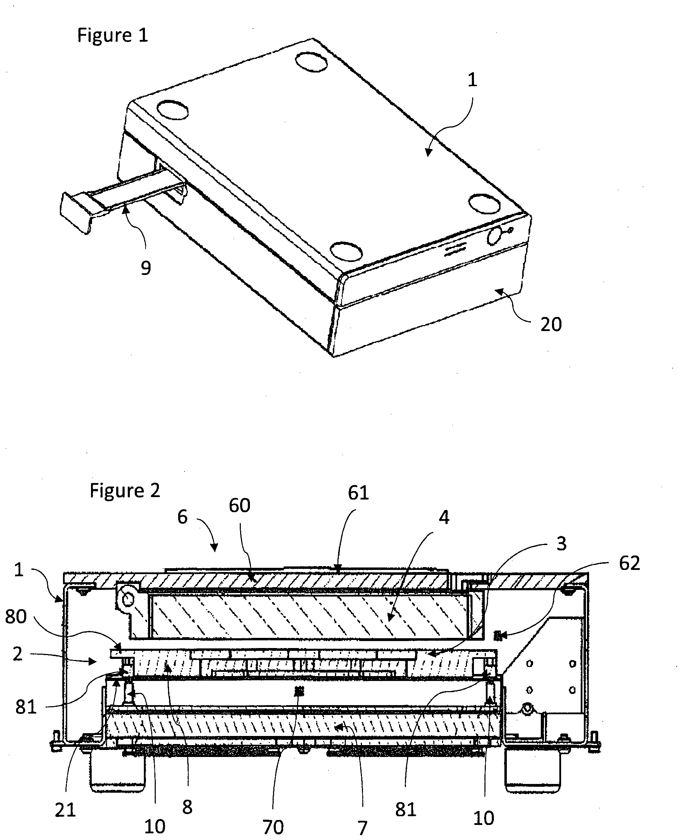

En référence à la figure 1, on peut voir un dispositif d'incubation et de détection de micro-organismes selon l'invention. Il se présente sous la forme d'un caisson 1, équipé d'un tiroir 2, dont n'est visible que la façade 20.

En référence à la figure 2, on peut voir que le dispositif selon l'invention comporte, intérieurement au caisson 1, une surface de détection 3 destinée à recevoir un réceptacle, fermé au moyen d'un couvercle transparent, lequel consiste généralement en une boîte de Petri, et contient le support de croissance des micro-organismes, comme une membrane ou un milieu gélosé.

La surface de détection 3 est associée au tiroir 2, en sorte que l'extraction du tiroir 2 permette le chargement et le déchargement de la surface de détection 3.

Le caisson 1 renferme également un système de détection 4, de type scanner linéaire, monté mobile au-dessus de la surface de détection 3 lorsque le tiroir 2 est fermé, en sorte de pouvoir balayer au moins en partie la surface de détection 3, et de préférence la totalité de ladite surface 3.

Le réceptacle est maintenu immobile au niveau de la surface de détection 3 pour permettre la lecture du résultat.

De préférence, plusieurs réceptacles sont disposés sur la surface de détection 3 du dispositif 1, de sorte à permettre une détection simultanée d'éventuels contaminants dans plusieurs échantillons différents.

Ainsi, le(s) réceptacle(s) comprenant un support de croissance reste(nt) immobile(s) sur la surface de détection 3, tandis que le système de détection 4 est mobile pour permettre la détection de la formation de colonies sur lesdits supports de croissance. Cela permet d'éviter un mouvement des supports pouvant être source d'erreurs sur les résultats obtenus.

Le système de détection 4 comporte préférentiellement au moins un capteur CCD (Charge-Coupled Device ou dispositif à transfert de charge) ou CMOS (Complementary Metal Oxide Semiconductor).

Le capteur CCD ou CMOS présente avantageusement une résolution supérieure ou égale à 2400 dpi (dot per inch ou point par pouce). Plus avantageusement encore, cette résolution est supérieure ou égale à 4800 dpi.

Une telle résolution permet la détection par imagerie des colonies présentes sur les supports de croissance, géloses ou membranes, positionnés dans les réceptacles, lorsque lesdites colonies présentent un diamètre inférieur ou égal à 100µm, voire inférieur à 50µm, et plus préférentiellement encore inférieur ou égal à 30µm, par un grandissement utile supérieur ou égal à 60.

Le système de détection 4 permet avantageusement une prise d'image à intervalles de temps réguliers.

Le capteur CCD ou CMOS du système de détection 4 est associé à un système optique composé d'au moins un éclairage et d'au moins une optique, par exemple de type lentille. De préférence, le système optique comporte également au moins un miroir.

Le dispositif d'incubation et de détection selon l'invention comprend des moyens de chauffage 6 disposés dans la partie supérieure du caisson 1, au-dessus de la surface de détection 3, et qui se composent, non limitativement, d'une plaque d'aluminium 60 associée à une résistance électrique 61. Ces moyens de chauffage 6 sont bien entendu destinés à maintenir à l'intérieur du caisson 1, la température d'incubation souhaitée.

Le dispositif d'incubation et de détection selon l'invention comprend également un système de prévention formation de condensation. En effet, comme il a déjà été évoqué ci-dessus la formation de condensation perturbe la lecture de la surface de la gélose ou de la membrane et ne permet donc pas d'obtenir des résultats satisfaisants.

Il est donc particulièrement important de résoudre ce problème de formation de la condensation, qui empêche une lecture correcte des échantillons et peut fausser les résultats.

A cet effet, le système de prévention de formation de condensation comporte, des moyens 7 de refroidissement, disposés dans la partie inférieure du caisson 1, sous le tiroir 2 et donc sous la surface de détection qui porte le ou les réceptacles.

On notera que ces moyens 7 de refroidissement peuvent consister, de préférence, en des modules thermoélectriques à effet Peltier.

Le système de prévention de formation de condensation comporte également un premier capteur de température 62 qui est positionné au-dessus du ou des réceptacles, et un second capteur de température 70, disposé en-dessous de la surface de détection 3, et donc en dessous du ou des réceptacles.

Le système de prévention de formation de condensation est complété par des moyens de gestion qui sont aptes à collecter et analyser les données ou informations recueillies par le premier capteur 62 et le second capteur 70, et aptes à commander les moyens de chauffage 6 et de refroidissement 7 pour, d'une part, générer la température d'incubation souhaitée et pour, d'autre part, appliquer en permanence un gradient de température à la surface du ou des réceptacles.

Un moyen privilégié de mesure de la température est obtenu en utilisant une thermopile permettant de mesurer sans contact, la température de surface du réceptacle.

L'application du gradient de température va permettre, de manière particulièrement avantageuse, d'éviter la formation de gouttelettes d'eau sur la surface interne du couvercle transparent du réceptacle. La lecture de la croissance des colonies sur le milieu gélosé ou sur la membrane s'en trouvera facilitée.

Dans un mode de réalisation avantageux, le gradient de température appliqué, au moyen du système de prévention formation de condensation, est d'au moins 0,10°C.

Cela signifie que la température appliquée au niveau du couvercle du réceptacle, c'est-à-dire au-dessus dudit réceptacle, est supérieure d'au moins 0,10°C à la température qui est appliquée au niveau du fond du réceptacle, autrement dit en-dessous de ce dernier.

De préférence, ce gradient est compris entre 0.10 et 1°C et, plus préférentiellement encore, ce gradient est compris entre 0,50 et 1°C.

Un tel gradient est particulièrement optimal pour éviter la formation de condensation sur le couvercle du réceptacle.

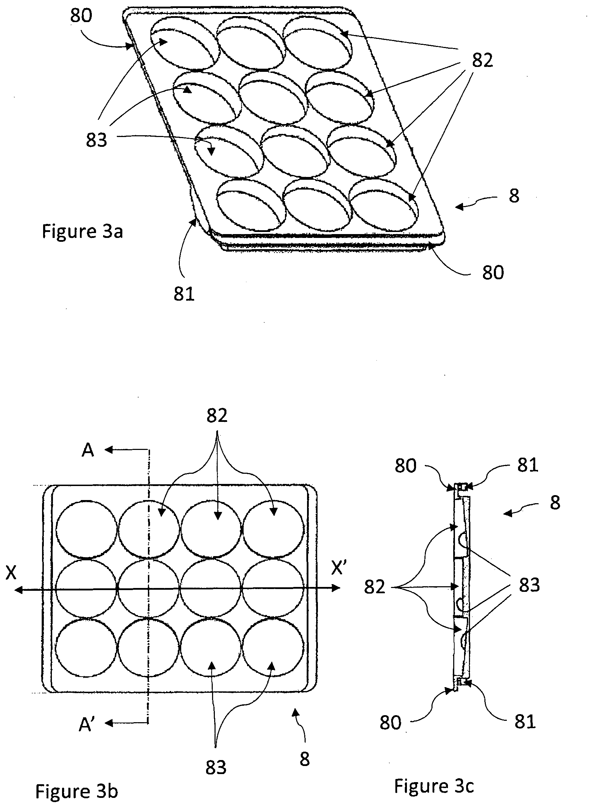

En référence également aux figures 3a, 3b et 3c, on peut voir un plateau 8 qui constitue la surface de détection destinée à recevoir les réceptacles, non représentés.

Ce plateau 8 est conçu amovible par rapport au tiroir 2. Ainsi, le tiroir 2 comporte un cadre 21, visible partiellement sur la figure 2, sur lequel le plateau 8 peut reposer par son bord périphérique 80, ou un partie de ce dernier, lorsque le tiroir 2 et maintenu extrait du caisson 1.

Le plateau 8 comporte également, inférieurement et en périphérie, des cales 81, destinées à coopérer avec des butées réglables 10, de type à vis par exemple, que comporte le caisson 1 en dessous de l'espace d'évolution du tiroir 2.

En fonctionnement, lors de la fermeture du tiroir 2, les cales 81 viennent prendre appui sur les butées réglables 10, et y demeurent pendant l'incubation et les détections, le plateau 8 étant alors désolidarisé du tiroir 2. Le réglage des butées permet un positionnement précis du plateau 8 et donc des réceptacles qu'il porte, par rapport au système de détection 4, et cela indépendamment du tiroir 2.

Sur les figures 3a, 3b et 3c, on peut voir que le plateau 8 comporte des emplacements 82, en l'occurrence au nombre de douze, chacun destiné à recevoir un réceptacle, non représenté.

Ces emplacements 82 consistent chacun en un logement en creux permettant de caler le réceptacle.

En référence plus particulièrement à la figure 3c, on peut voir que selon la position de l'emplacement 82 sur le plateau 8, le fond 83 de l'emplacement 82 présente une inclinaison particulière par rapport au plan général du plateau 8. Ainsi, les emplacements 82 médian, par rapport à l'axe XX' de déplacement du moyen de détection par rapport au plateau 8, présentent un fond 83 parallèle au plan général du plateau 8, tandis que les emplacements 82 latéraux présentent un fond 83 incliné en direction de la ligne médiane.

Ces configurations permettent d'éviter les aberrations optiques susceptibles d'empêcher ou de perturber une détection des colonies.

Enfin, on notera, comme cela est visible sur la figure 1, que le système de détection, de type scanner, du dispositif d'incubation et de détection selon l'invention comporte un système de calibration, présentant la particularité d'être un élément 9 extractible du caisson 1, et de préférence amovible, dans un but de nettoyage et/ou de remplacement.

Le dispositif d'incubation et de détection selon l'invention, présente l'avantage, par rapport à ceux de l'état de la technique, d'une compacité et une simplicité de fabrication.

Il offre également l'avantage d'une possible modularité, puisqu'il peut être dimensionné pour le traitement, d'un nombre variable de réceptacle, en adaptant le plateau 8 par exemple, mais aussi parce que plusieurs caissons 1 peuvent être superposés et/ou juxtaposés, sans pour cela représenter un encombrement important.

Ces avantages sont essentiellement dus au système de détection utilisé, ainsi qu'à la manière dont il est mis en œuvre. Ce système de détection ne pourrait toutefois pas être aussi performant sans l'une ou l'autre, ou l'ensemble, des différentes caractéristiques énoncées ci-dessus.

## Revendications

1. Dispositif d'incubation et de détection, rapide et sans mesure de fluorescence, de colonies issues de la multiplication de micro-organismes présents dans un échantillon à tester, comprenant :
- des moyens de chauffage (6),
- une surface de détection (3) sur laquelle est disposé, immobile, au moins un réceptacle fermé d'un couvercle transparent dans lequel est placé un support de croissance des micro-organismes sous forme de colonies, ce support étant de type membrane ou milieu gélose,
- un système de détection (4), comprenant un système optique permettant de visualiser lesdites colonies,
**caractérisé en ce qu'**il comporte de plus un système de prévention de la formation de condensation dans ledit réceptacle, qui comprend un moyen de régulation de la température composé :
- d'un premier capteur de température (62) opérant au-dessus dudit réceptacle,
- d'un second capteur de température (70) opérant en dessous dudit réceptacle,
- dudit moyen de chauffage (6), qui agit au-dessus dudit réceptacle,
- d'un moyen de refroidissement (7) agissant en-dessous dudit réceptacle, et
- des moyens de gestion aptes à collecter et analyser les informations recueillies par lesdits moyens capteurs, et à commander lesdits moyens de chauffage (6) et de refroidissement (7) en sorte de générer la température d'incubation d'une part souhaitée, et d'appliquer en permanence un gradient de température à la surface dudit réceptacle d'autre part.

2. Dispositif selon la revendication 1, **caractérisé en ce que** le système de détection est de type scanner linéaire, monté mobile et à plat pour balayer en tout ou partie ladite surface, comportant au moins un capteur CCD ou CMOS auquel est associé un système optique composé d'au moins un éclairage et d'au moins une optique, de type lentille.

3. Dispositif selon la revendication 1 ou la revendication 2, **caractérisé en ce qu'**il comporte des moyens d'ajustement de la hauteur du ou des réceptacles, et donc du ou des supports de croissance, par rapport au système optique.

4. Dispositif selon l'une quelconque des revendications 1 à 3, **caractérisé en ce qu'**il comporte des moyens de correction automatique de la distance qui sépare le système optique du ou des réceptacles, et donc du ou des supports de croissance.

5. Dispositif selon l'une quelconque des revendications 2 à 4, **caractérisé en ce que** la surface de détection consiste en un tiroir (2), monté coulissant dans ledit dispositif, apte à passer d'une position ouverte, pour recevoir le ou les réceptacles fermés, à une position fermée, dans laquelle peut être réalisé un balayage du ou desdits réceptacles et donc du ou des supports de croissance par le système de détection, lesdits supports étant disposés directement dans ledit tiroir (2) .

6. Dispositif selon la revendication 5, **caractérisé en ce que** le ou les réceptacles sont disposés dans le tiroir (2) au travers d'un plateau amovible (8).

7. Dispositif selon la revendication 6, **caractérisé en ce que** le plateau amovible (8) est muni en périphérie et inférieurement de cales (81) prévues aptes, lorsque le tiroir (2) est en position fermée, à coopérer avec des butées de profondeur réglables (10) que comporte intérieurement ledit dispositif, en sorte d'amener, par soulèvement, et de maintenir ledit plateau (8), et donc le ou les réceptacles qu'il porte, à la bonne distance du système optique.

8. Dispositif selon l'une quelconque des revendications 1 à 7, **caractérisé en ce que** la surface de détection sur laquelle est disposé au moins un réceptacle, est configurée pour donner à chacun des réceptacles une inclinaison par rapport au système optique.

9. Dispositif selon l'une quelconque des revendications 1 à 8, **caractérisé en ce que** le système optique comporte un système de calibration qui consiste en un élément (9) amovible dans un but de nettoyage et/ou de remplacement.

10. Dispositif selon l'une quelconque des revendications 1 à 9, **caractérisé en ce que** la mesure de température est réalisée au moyen d'une thermopile.

## Patentansprüche

1. Vorrichtung zur schnellen Inkubation und Erfassung ohne Fluoreszenzmessung von Kolonien, die aus der Vermehrung von in einer Testprobe vorhandenen Mikroorganismen stammen, umfassend:
- Heizmittel (6),
- eine Erfassungsfläche (3), auf der mindestens ein mit einem durchsichtigen Deckel geschlossener Behälter unbeweglich angeordnet ist, in dem ein Wachstumsmedium für Mikroorganismen in Form von Kolonien eingebracht ist, wobei dieses Medium der Art Membran oder Agar-Gel ist,
- ein Erfassungssystem (4), das ein optisches System umfasst, das es ermöglicht, die besagten Kolonien sichtbar zu machen,
**dadurch gekennzeichnet, dass** sie außerdem ein System zum Verhindern der Bildung von Kondensation in dem besagten Behälter umfasst, das ein Mittel zum Regulieren der Temperatur umfasst, das aus Folgendem besteht:
- einem ersten Temperatursensor (62), der oberhalb des besagten Behälters betrieben wird,
- einem zweiten Temperatursensor (70), der unterhalb des besagten Behälters betrieben wird,
- dem besagten Heizmittel (6), das oberhalb des besagten Behälters wirkt,
- einem Kühlmittel (7), das unterhalb des besagten Behälters wirkt, und
- Verwaltungsmitteln, die in der Lage sind, die von den Sensormitteln gesammelten Informationen zu sammeln und zu analysieren und die Heiz- (6) und Kühlmittel (7) so zu steuern, dass einerseits die gewünschte Inkubationstemperatur erzeugt wird und andererseits dauerhaft ein Temperaturgradient an die Oberfläche des besagten Behälters angelegt wird.

2. Vorrichtung nach Anspruch 1, **dadurch gekennzeichnet, dass** das Erfassungssystem der Art Linearscanner ist, der beweglich und flach montiert wird, um die besagte Oberfläche völlig oder teilweise abzutasten, umfassend mindestens einen CCD- oder CMOS-Sensor, dem ein optisches System zugeordnet ist, das aus mindestens einer Beleuchtung und mindestens einem optischen System der Art Linse besteht.

3. Vorrichtung nach Anspruch 1 oder Anspruch 2, **dadurch gekennzeichnet, dass** sie Mittel zum Einstellen der Höhe des bzw. der Behälter und also des bzw. der Wachstumsmedien relativ zum optischen System umfasst.

4. Vorrichtung nach irgendeinem der Ansprüche 1 bis 3, **dadurch gekennzeichnet, dass** sie Mittel zum automatischen Korrigieren des Abstands, der das optische System von dem bzw. den Behältern und also von dem bzw. den Wachstumsmedien trennt, umfasst.

5. Vorrichtung nach irgendeinem der Ansprüche 2 bis 4, **dadurch gekennzeichnet, dass** die Erfassungsfläche aus einer Schublade (2) besteht, die verschiebbar in der besagten Vorrichtung montiert und geeignet ist, aus einer offenen Position zum Aufnehmen des bzw. der geschlossenen Behälter in eine geschlossene Position zu gehen, in der eine Abtastung des bzw. der besagten Behälter und also des bzw. der Wachstumsmedien durch das Erfassungssystem durchgeführt werden kann, wobei die besagten Medien direkt in der Schublade (2) angeordnet sind.

6. Vorrichtung nach Anspruch 5, **dadurch gekennzeichnet, dass** der bzw. die Behälter mittels einer abnehmbaren Platte (8) in der Schublade (2) angeordnet sind.

7. Vorrichtung nach Anspruch 6, **dadurch gekennzeichnet, dass** die abnehmbare Platte (8) am Umfang und an der unteren Seite mit Keilen (81) versehen ist, die geeignet vorgesehen sind, wenn sich die Schublade (2) in der geschlossenen Position befindet, so mit einstellbaren Tiefenanschlägen zusammenzuwirken, welche die besagte Vorrichtung innen umfasst, dass die besagte Platte (8) und also der bzw. die Behälter, die sie trägt, durch Anheben auf den richtigen Abstand vom optischen System gebracht und gehalten werden.

8. Vorrichtung nach irgendeinem der Ansprüche 1 bis 7, **dadurch gekennzeichnet, dass** die Erfassungsfläche, auf der mindestens ein Behälter angeordnet ist, konfiguriert ist, um jedem der Behälter eine Neigung relativ zum optischen System zu verleihen.

9. Vorrichtung nach einem der Ansprüche 1 bis 8, **dadurch gekennzeichnet, dass** das optische System ein Kalibrierungssystem umfasst, das aus einem Element (9) besteht, das zum Zweck der Reinigung und/oder des Ersetzens abnehmbar ist.

10. Vorrichtung nach irgendeinem der Ansprüche 1 bis 9, **dadurch gekennzeichnet, dass** die Temperaturmessung mittels einer Thermosäule durchgeführt wird.

## Claims

1. Detection and incubation device for high-speed detection of a colony without fluorescence measurement, said colony being obtained from growth of a microorganism which exists in a sample to be examined, said device comprising :
- a heating means (6),
- a detection surface (3) where at least one container closed with transparent covering has been placed in a fixed position and where an organism growth culture medium of a film or an agar type of a form of a colony is arranged,
- a detection system (4) comprising an optical system enabling visualizing the colony,
**characterised in that** said device further comprises a prevention system configured to prevent formation of dew condensation in the container, said prevention system comprising temperature adjusting means including:
- a first temperature sensor (62) configured to operate in the upper part of the container,
- a second temperature sensor (70) configured to operate in a lower part of the container,
- the heating means (6) configured to act in the upper part of the container,
- a cooling means (7) configured to act in a lower part of the container,
- a control means enabling collecting and analysing information collected by the sensors, and configured to control the heating means (6) and the cooling means (7) in order to generate a desired incubation temperature and to apply permanently a temperature gradient to the surface of the container.

2. Detection and incubation device according to claim 1, wherein the detection system (4) is a linear scanner, arranged movable and laid flat in order to scan all or some of the detection surface, comprises at least one CCD or CMOS sensor at which is associated the optical system comprising at least one lighting and at least one optical element such as a lens.

3. Detection and incubation device according to claim 1 or 2, comprising adjusting means configured to adjust height of one or more containers and therefore of one or more growth culture media, compared to the optical system.

4. Detection and incubation device according to claims 1 to 3, comprising correction means configured to correct automatically distance between the optical system and one or more containers therefore one or more growth culture media.

5. Detection and incubation device according to claims 2 to 4, wherein the detection surface is a drawer (2), arranged slidably inside the detection and incubation device, said detection surface being able to move from an open position for receiving one or more closed containers, to a closed position, wherein a scan of the one or more containers can be performed, therefore a scan of the one or more growth culture media can be performed, by the detection system (4), the culture media being arranged directly inside the drawer (2).

6. Detection and incubation device according to claim 5, wherein the one or more containers are arranged inside the drawer via a removable tray (8).

7. Detection and incubation device according to claim 6, wherein the removable tray (8) is equipped of block elements (81) in its periphery and below, said block elements being configured to cooperate with adjustable depth stop elements (10) when the drawer is in closed position, said adjustable depth stop elements (10) being included inside the detection and incubation device, in order to bring by lifting and to maintain the removable tray and therefore the one or more containers, at a right distance of the optical system.

8. Detection and incubation device according to claims 1 to 7, wherein the detection surface on which is arranged at least one container, is configured to tilt each container compared to the optical system.

9. Detection and incubation device according to claims 1 to 8, wherein the optical system comprises a calibration system which is a removable element (9) in order to be cleaned and/or be replaced.

10. Detection and incubation device according to claims 1 to 9, wherein the temperature measurement is performed by a thermopile.
